# EUROPEAN PATENT SPECIFICATION

(11) **EP 3 227 403 B1**
(45) Date of publication and mention of the grant of the patent: **25.02.2026**
(21) Application number: 15866062.1
(22) Date of filing: 04.12.2015
(51) Int. Cl.: A61K 8/97, C09K 15/34, A61K 36/899, A61P 31/00, A61P 39/06, A61Q 17/00

(54) **ANTIOXIDANT AND/OR ANTIMICROBIAL COMPOSITION BASED ON PALM OIL**
ANTIOXIDATIVE UND/ODER ANTIMIKROBIELLE ZUSAMMENSETZUNG AUF BASIS VON PALMÖL
COMPOSITION ANTIOXYDANTE ET/OU ANTIMICROBIENNE À BASE D'HUILE DE PALME

(30) Priority: 04.12.2014 MY PI2014703657
(43) Date of publication of application: 11.10.2017
(73) Proprietor: Universiti Putra Malaysia, 43400 Serdang, Selangor (MY)
(72) Inventor: LAI, Oi Ming, Serdang Selangor 43400 (MY); TAN, Chin Ping, Serdang Selangor 43400 (MY); LAI, Wee Ting, Serdang Selangor 43400 (MY); NICHOLAS, Khong Mun Hoe, Serdang Selangor 43400 (MY)
(74) Representative: Carpmaels & Ransford LLP
(86) International application number: PCT/MY2015/050150
(87) International publication number: WO 2016/089200

(56) References cited:
- EP-A1- 2 505 185
- EP-B1- 0 349 138
- WO-A1-00/44375
- WO-A1-2008/142433
- WO-A1-2010/036578
- JP-A- 2013 018 940
- JP-A- 2013 018 940
- JP-A- H08 182 465
- US-A1- 2004 241 254
- US-A1- 2010 074 963
- US-A1- 2010 279 907
- INTERNATIONAL JOURNAL OF BIOLOGY, vol. 3, no. 2, 2011, pages 153 - 161, XP055453214, ISSN: 1916-968X
- JOURNAL OF AGRICULTURAL AND FOOD CHEMISTRY, vol. 54, 2006, pages 3445 - 3453, XP009503678, ISSN: 0021-8561
- JOURNAL OF THE CHEMICAL SOCIETY OF PAKISTAN, vol. 33, no. 3, 2011, pages 343 - 350, XP009503679, ISSN: 0253-5106
- JOURNAL OF OIL PALM RESERCH, vol. 13, no. 2, 2002, pages 23 - 32, XP009503682, ISSN: 1511-2780
- EUROPEAN JOURNAL OF CLINICAL NUTRITION, vol. 55, 2001, pages 657 - 662, XP055453224, ISSN: 0954-3007

## Description

The present invention relates to compositions comprising red palm oil, or fractions thereof, wherein the red palm oil comprises carotenoids, vitamin E, sterols, phosphatides and total alcohols. More particularly, the present invention relates to a composition derived from red palm oil having antioxidant and/or antimicrobial activity, which can be incorporated as additives and/or preservatives in food, cosmetics, feed, toiletries, pharmaceuticals and medicines.

### BACKGROUND OF THE INVENTION

Spoilage and deterioration of active components can be attributed to (1) the attack by pathogens especially bacteria and molds, and/or (2) oxidation that causes destruction of essential biochemical compounds. Thus, the magnitude of active preservatives is very much as important as the quality of the product itself. Additives meant for product preservation and shelf life elongation have become an increasingly important component of the food, cosmetics, pharmaceutical and biomedical industries as international trades expand rapidly across the globe. Ideally, a good preservative is expected to be a non-toxic active capable of reducing or eliminating both of those causative agents. Actives exhibiting both antioxidative and antimicrobial properties would be beneficial to the industrial sectors to extend product shelf life, improve product safety, maintain product quality, reduce processing costs and increase the ability to distribute products globally in complex supply chains. Due to consumer concern about the use of synthetic additives, the market for synthetic bioactives is declining and being replaced with natural bioactives.

No food, feed, cosmetics and medicines could be marketed without the inclusion of any forms of preservative and/or stabilizing additives. Some familiar examples of the former class of food additives are sodium benzoate and benzoic acid; calcium, sodium propionate, and propionic acid; calcium, potassium, sodium sorbate, and sorbic acid; and sodium and potassium sulfite. Examples of the latter class of additives include calcium, sodium ascorbate, and ascorbic acid (vitamin C); butylated hydroxyanisole (BHA) and butylated hydroxytoluene (BHT); lecithin; and sodium and potassium sulfite and sulfur dioxide. A special class of additives that reduce oxidation is known as the sequestrants. Sequestrants are compounds that "capture" metallic ions, such as those of copper, iron, and nickel, and remove them from contact with foods. The removal of these ions helps preserve foods because in their free state they increase the rate at which oxidation of foods takes place. Some examples of sequestrants used as food preservatives are ethylenediamine-tetraacetic acid (EDTA), citric acid, sorbitol, and tartaric acid. Perhaps the most common chemical preservatives found in majority of industrial preservation operations used today is the parabens. They can be found in food shampoos, commercial moisturizers, shaving gels, persona lubricants, topical/parenteral pharmaceuticals, spray tanning solution, makeup and toothpaste. However, the usage of these chemical additives is becoming increasingly controversial. The benefits and safety of many artificial food additives (including preservatives) are the subject of debate among academics and regulators specializing in food science and toxicology. The parabens have been associated with cancer tumour growth (Harvey & Everett, 2004). There is also a growing concern to the possible toxicity of synthetic antioxidants such as BHA and BHT to human health (LevisNexis, 2002).

For a long time it was a common practice to add subtherapeutic amounts of antibiotics, such as tetracycline, to the feeds of livestock to promote growth and improve productivity. When antibiotic resistance in foodborne human pathogens was reported, this practice was either banned or voluntarily abandoned in many countries. The task of controlling the intestinal microflora in food animals, in the absence of antibiotics, is two-fold: First, to modulate the composition and number of commensal bacteria in the gastrointestinal tract so that it is as favorable as possible to the health and productivity of the animal; and second, to reduce asymptomatic intestinal colonization by pathogenic bacteria in the animals to lower the possibility of foodborne transmission to humans. Unfortunately, the knowledge of what constitutes a healthy, balanced intestinal microflora is still incomplete. This makes the task of favorably changing its composition difficult. However, unnecessary use of antibiotics has fuelled to the increasing incidence of drug-resistant pathogens that has drawn attentions of the industrial and scientific communities alike. It has thus become evident that there is urgent need for novel antibacterial drugs with broader spectrum, lesser side effects, and without cross-resistance to antibiotics in use. In addition, for fungi and protozoa, current chemotherapeutic options are very limited and far from ideal, especially for undesirable side effects or toxicity. Modulation by means of natural feed supplements has been successfully practised for a number of years, the most important being probiotics, prebiotics, bacteriocins, organic acids, enzymes, bioactive phytochemicals, antimicrobial peptides, lipids and bacteriophages.

In fact, during the last few years, the antimicrobial effect of plant oils and extracts has formed the basis of many applications, including raw and processed food preservation, pharmaceuticals, alternative medicine and natural therapies. Essential oils, derived from aromatic medicinal plants (e.g., fennel, peppermint, thyme and lavender) and containing mixtures of volatile substances, such as monoterpenes, sesquiterpenes and/or phenylpropanoids, have been reported to be active on Gram-positive and Gram-negative bacteria and on yeasts, fungi and viruses. Helicobacter pylori was susceptible to different essential oils, in particular carrot (*Daucus carota*) seed oil and *Mycoplasma pneumonia* to tea tree (*Melaleuca alternifolia*) oil, which seems to affect the intracellular cytoskeleton structure. Essential oil mixtures, primarily composed of terpenoids and aromatic and aliphatic constituents have been studied for antimicrobial activity also against caries-related bacteria. Some components acts as inhibitors of bacterial growth, some as inhibitors of exopolysaccharide synthesis and others inhibit bacterial adherence. Yet, most studies of oil antimicrobials focused on essential oils. Very little attention has been given to the antimicrobial effects of edible oils.

Palm oil is an edible vegetable oil derived from the mesocarp (reddish pulp) of the fruit of the oil palms, primarily the African oil palm *Elaeis guineensis,* and to a lesser extent from the American oil palm *Elaeis oleifera* and the maripa palm *Attalea maripa.* Palm oil is naturally reddish in color because of high beta-carotene content. Palm oil is, like all fats, composed of fatty acids, esterified with glycerol. It is high in saturated fatty acids. Palm oil gives its name to the 16-carbon saturated fatty acid palmitic acid. Monounsaturated oleic acid is also a constituent of palm oil. Unrefined palm oil is a large natural source of tocotrienol, part of the vitamin E family

In view of the above, it is advantageous to provide a composition based on red palm oil having antioxidant and/or antimicrobial activity, which can be incorporated as additives and/or preservatives in food, cosmetics, feed, toiletries, pharmaceuticals and medicines. Red palm oil obtains its name from its characteristic dark red color, which comes from carotenes, such as alpha-carotene, beta-carotene and lycopene, the same nutrients that give tomatoes, carrots and other fruits and vegetables their rich colors. Red palm oil contains at least 10 other carotenes, along with tocopherols and tocotrienols (members of the vitamin E family), CoQ10, phytosterols, and glycolipids.

JP 2013 018940 A discloses a solid soap and a method for producing the same, and more particularly to a novel solid soap which is said to be gentle to the skin and also has a cosmetic effect and a method for efficiently producing the solid soap.

WO 2010/036578 A1 discloses the novel composition of red palm oil and fish oil. When applied topically to mammalian skin, this mixture is said to strengthen the acid mantle and reduce inflammation of the skin. Fish oil is high in very long chain omega 3 & 6 fatty acids but low in Cm omega 3 & 6 fatty acid. The novel mixture can also be described as a mixture of red palm oil and very long chain (>C₁₈) omega 3 & 6 fatty acids.

WO 2008/142433 A1 discloses a process for producing a composition comprising tocopherols and tocotrienols and to the compositions thereby produced.

US 2004/241254 A1 discloses cosmeceutical formulations and methods for improving the appearance, feel, comfort, or biological functioning of the skin. More specifically, the invention relates to cosmeceutical formulations comprising palm oil and red palm olein and its application to the skin for cosmetic reasons and for the treatment of various physical, environmental, and medical skin conditions.

EP 2 505 185 A1 discloses a composition which is said to provide improved tanning effect for human skin and the use of the composition in dermatological and cosmetic applications.

US 2010/279907 A1 discloses hygiene products.

EP 0 349 138 B1 discloses a process for the recovery of carotenes comprising the steps of transesterification of oil containing carotenes and vacuum distillation, and characterised in that 0.1% to 50% of edible oil is added to the transesterified oil containing carotenes or solution containing carotenes and that the resulting mixture is subjected to a pressure of less than 0.060 Torr and a temperature of less than 200 degrees Celsius.

### SUMMARY OF THE INVENTION

The invention is described in the appended claims.

The present inventors have discovered, surprisingly, a composition containing red palm oil or one or more fractions thereof having antioxidant and/or antimicrobial activity, paving the way to a variety of applications in the food, cosmetic, feed, toiletry, pharmaceutical and medicine field.

It is therefore an object of the present invention to provide an antioxidant and/or antimicrobial composition containing red palm oil or one or more fractions thereof. The red palm oil may be extracted from the mesocarp (reddish pulp) of the fruit of the oil palms, primarily the African oil palm *Elaeis guineensis* and to a lesser extent from the American oil palm *Elaeis oleifera* and the maripa palm *Attalea maripa.*

The red palm oil may be obtained by any known method, such as molecular distillation of the mesocarp of the fruit of oil palm. The red palm oil may be further fractionated in order to extract a variety of red palm oil that is free of undesirable alkaloids.

The present invention relates more particularly to a composition comprising red palm oil in the form of a fraction consisting of red palm oil concentrate obtained by molecular distillation of the oil.

Advantageously, the quantity by weight of the unsaponifiable fraction in the red palm oil concentrate is about 1% to about 5%.

The inventors have discovered that red palm oil has a particularly high content of polyphenol, β-carotene, tocopherol and tocotrienol derivatives and it is known that these derivatives contribute to the oxidation stability of a composition containing them. Advantageously, the object of the invention is an antioxidant and/or antimicrobial composition that contains sterols, higher aliphatic alcohols, pigments, and hydrocarbons. Compositions of the invention comprise carotenoids, vitamin E, sterols, phosphatides, and total alcohols derivatives extracted from red palm oil. In the present invention, the content of micronutrients are carotenoids in the amount of 500-700 ppm, vitamin E in the amount of 500-800 ppm, sterols in the amount of up to 300 ppm, phosphatides in the amount of 500-100 ppm and total alcohols in the amount of 0-800 ppm.

The present invention also provides a composition in which the red palm oil or its fractions are in the form of a mixture with coconut oil or one or more of its fractions. In this aspect, the coconut oil fractions may also be a concentrate obtained by molecular distillation of the oil or alternatively an unsaponifiable fraction contained in such a concentrate. The inventors have surprisingly established that the coconut oil or its fractions could be advantageously used in combination with red palm oil or its fractions.

In a preferred embodiment, when the two types of oil are present in a composition according to the present invention, the red palm oil is in the form of a mixture with a coconut oil concentrate.

Preferably, the quantities by weight of concentrate of red palm oil and red palm oil vary respectively between about 10% and about 90% and between about 90% and about 10% so that the quantities of these oils are 100% in total.

According to a preferred composition, the quantities by weight of the concentrate of red palm oil and of coconut oil are 70% and 30% respectively.

In one aspect, the present invention can be used as an additive and/or preservative, in particular as food or animal feed additive and/or preservative with high antioxidant and antimicrobial activity for improving food stability, shelf life and inhibiting or preventing the formation of free radicals and food spoilage organisms.

In another aspect, the present invention can also be used as a cosmetic, in particular as an antioxidant, anti-free radical agent and agent for protecting skin damage, especially oxidative damage. With the activities shown by the composition of the present invention, it is possible to use the composition for cosmetic or pharmaceutical purposes, for example for photoprotection against actinic aging and/or for protecting the skin against oxidative attacks including pollution.

In also another aspect, the present invention provides a composition according to the present invention for use in therapy, especially for use as a dermatological, in particular as an agent for preventing or treating the effects of UVA and/or UVB radiation on the skin, as a pharmaceutical product for treating and preventing oxidative/free radical effects on the skin, or as an agent for protecting the skin at cellular or DNA level against damage, especially oxidative damage.

In yet another aspect there is provided an additive, preservative, cosmetic or pharmaceutical composition comprising a composition according to the present invention, preferably in combination with a physiologically acceptable carrier.

The additive, preservative, cosmetic or pharmaceutical composition according to the present invention are capable of being used as an additive and/or preservative in food products for improving food stability, shelf life and inhibiting or preventing the formation of free radicals and food spoilage organisms, a natural preservative in food products, an anti-sun product for protecting against ultra violet radiation or infrared radiation, a toning cream, nutritive cream, anti-wrinkle cream, protective cream, contour of the lips and the eyes and protective lipstick. For these uses, the additive, preservative, cosmetic or pharmaceutical composition are formulated tor a topical use, particularly in the form of creams, emulsions, ointments, sticks or gels.

The formulated additive, preservative, cosmetic or pharmaceutical composition includes, but not limited to, emulsions, micro- and nano- modifications, microencapsulation, nanoencapsulation, control release, liposomes, esterification and interesterifications, as well as and mixtures and/or blends which exhibit synergistic effects with phytochemicals and other oils such as virgin coconut oil, rapeseed oil, olive oil, tea tree oil, or any essential oil and edible oil.

The additive, preservative, cosmetic or pharmaceutical composition according to the present invention may have a total content by weight of red palm oil or its fractions and coconut oil or its fractions of the order of 5%-100%.

The antioxidant activity of the compositions according to the present invention, which is shown by the oxidation-stability of the red palm oil and of the corresponding concentrate, is particularly advantageous because it offers possibilities of additional uses as a dietary supplement taking advantage of the antioxidant activity.

Also disclosed but not claimed is a process for preparing the composition as described above. Different variations may be practised depending on the compositions. For example, the process may include mixing red palm oil and coconut oil, which is followed by molecular distillation of the mixture.

### BRIEF DESCRIPTION OF THE DRAWINGS

Figure 1 is a graph showing comparison of colony forming unit (cfu/ml) of noodle of negative control, noodle with red palm oil (RPO) and noodle with virgin coconut oil (VCO);
Figure 2 shows inhibition of different oil on the growth of *Bacillus cereus* (left: VCO; middle; control: distilled water; right: RPO);
Figure 3 shows inhibition of different oil on the growth of *Candida albicans* (left: VCO; middle; control: distilled water; right: RPO);
Figure 4 shows inhibition of different oil on the growth of *Propionibacterium acnes* (left: VCO; middle; control: distilled water; right: RPO); and
Figure 5 shows inhibition of different oil on the growth of *Proteus mirabilis* (left: VCO; middle; control: distilled water; right: RPO).

### DETAILED DESCRIPTION OF THE INVENTION

The present invention will now be described in further detail by way of non-limiting examples.

### EXAMPLE 1

### Preparation of Red Palm Oil

### Neutralization

The equipment used for neutralization was FT66, Neutralizer/Washer/Bleacher (Armfield, Ringwood, Hampshire, UK). The main parts of this equipment are reactor vessel, agitator drive, bleaching earth hopper, reagent tank, pressure leaf filter, liquid ring vacuum pump, filter pump and control console. The reactor vessel is designed to process 25 L per batch. The internal parts of this reactor vessel include: agitator paddle, baffle arrangement, electrical heating element and cooling coil.

Twenty kilograms of crude palm oil (CPO) was charged into the neutralizer by applying vacuum (70 mmHg). The CPO was heated under vacuum with gentle agitation to 80°C. Calculated amount of NaOH (115 g) was dissolved in required quantity (1 L) of distilled water and heated to 80°C in the reagent vessel and slowly admitted to the reactor vessel and mixed well under slow stirring maintaining vacuum. After 10 min, the stirring was stopped and the oil was cooled to 70°C using chilled water and vacuum released at 70°C and further cooled to 48 to 50°C. After settling for a period of 2 hours, the soap stock was removed from the bottom of the vessel. The separation of the soap stock was completed before the temperature dropped below 48°C. After soap separation, the oil was washed free of alkali and soap using hot water at 80°C under vacuum. Four to five washings were required with oil to water ratio of 7:1 for each washing.

### Crystallization

Crystallizer of 50 kg capacity (M/s Thermosystems, Trivandrum, India) was used for crystallization. This equipment is a vertical, cylindrical, jacketed stainless steel vessel with scraped surface blade type of agitator, with a variable speed drive. The agitator is designed to scrape the inner surface of the crystallizer so as to avoid crystal accumulation and consequent heat transfer problems. The entire heating and cooling system is controlled by PID (Proportional Integral Derivative) controller having facility to pre-program the heating and cooling rates as per the requirement. Thermocouple probes are provided at two positions (top and bottom) through the agitator shaft and connected to the PID controller so as to maintain the temperature as per the heating/cooling programme uniformly throughout the mass.

Neutralized palm oil (NPO) was fed into the crystallizer and heated to 70°C by admitting steam into the jacket, over a period of 60 min and held at 70°C for 15 min to destroy any crystal. Oil was then cooled to 25°C at different cooling rates of 15, 17, 20, 25, 30 and 35°C per hour by admitting chilled water into the jacket. Different rate of cooling was employed here, to arrive at the optimum DT at which yield of crystals was maximum. The heating and cooling, at the set rate was controlled by PID through solenoid valve. When the temperature of the oil reached 25°C the crystallized mass was taken out for separation.

### Filtration

The crystallized palm oil was then separated into palm stearin (PS) and olein using rotary drum vacuum filter (M/s Thermosystems, Trivandrum, India), which has a rotating drum on which the filter medium, in this case ordinary 100% polyester cloth, is used. The drum is rotated by means of a variable speed drive. Rotating drum, partially submerged in the oil, uses vacuum to draw the oil through the filter of the drum. The olein fraction was separated and the filtrate discharged to a holding tank. The PS cake on the rotating drum was scraped off by knife blade continuously from the outer surface of the drum and collected in the receiving trough. The liquid palm olein was then subjected to deodorization.

### Deodorization

Deodorization was carried out in a FT 68 Deodorizer (Armfield, Ringwood, Hampshire, UK). It is a cylindrical stainless steel vessel of 30 L capacity, which contains a specially designed baffle system to sparge steam that promotes efficient mixing of the oil and live steam. The vessel is provided with electrical heating element and a stainless steel internal cooling coil. The high vacuum required for the process is achieved by combination of a multi-stage liquid ring vacuum pump in series, and an efficient steam ejector. Twenty-five kilograms of WPOn was taken for deodorization in each batch. The deodorization vessel was evacuated first to 1 mmHg by operating the water ring vacuum pump and steam ejector. After reaching the vacuum to 1 mmHg, the oil was charged into the deodorization vessel using vacuum. The deodorization was conducted at 130, 140 and 150 C for 2 h. Different deodorization temperatures were used here to arrive at the minimal temperature at which retention of carotene was maximum without any unpleasant odor. The oil was heated to 70°C using electric heating followed by live steam sparging at 0.35 kg h⁻¹. Further heating to set temperature was achieved by electric power and live steam sparging. Samples were withdrawn every half an hour through the sampling valves to monitor the progress of deodorization for each experiment.

### EXAMPLE 2

### Preparation of Red Palm Oil Unsaponifiable Phytonutrients Concentrate

2 grams of palm phytonutrients concentrate was saponified at 60°C with 5 mL of 50% (w/w) potassium hydroxide solution and 20 mL ethanol. The mixture was refluxed in the dark for 1 hour. The reacted mixture was then extracted 5 times with hexane until a colourless organic layer was obtained. The extracted organic layer was then washed with distilled water until the waste water was tested neutral in phenolphthalein. Excess solvent was evaporated and the sample was then stored in a cold, dry place.

### EXAMPLE 3

### Preparation of Mixture of a Red Palm Oil and Coconut Oil

Red Palm Olein was mixed with virgin coconut oil in any proportion under slightly heated temperature ranging from 45°C to 55°C.

### EXAMPLE 4

### Food-Based Formulation

The method of preparing salted noodles was modified from Park and Baik (2000). 300 g of flour was prepared and 2% of salt was added; this was mixed with water at low speeds for 3 min, then rolled into a 3-mm thick sheet of dough. Folding and sheeting were repeated twice more. The dough sheet was allowed to rest for 1 h and then put through the sheeting rolls three times a progressively decreasing roll gaps of 2.60, 2.33, 2.00 mm. From this sheet, the noodle strands were cut and the prepared noodles were placed in plastic bags. The experiment was repeated by adding in 30 g of red palm olein and virgin coconut oil.

### EXAMPLE 5

### Multi-purpose Film Formulation

Alginate-based edible films were prepared by modification of the method used by Pavlath et al., 1999. Sodium alginate (1 g) was dissolved into 100 mL of distilled water and rotary shaking was done concurrently. Since the alginate film was brittle, 0.4 ml of glycerol was added into the edible film solution. Red palm olein and virgin coconut oil were then incorporated into the film solution at concentration 1 v/v of edible film solution. The solutions were cast into 90mm dia. x 15mm petri dish by oven drying at 40°C for 20-24 hours. The dry films obtained were peeled off and stored for evaluation

### EXAMPLE 6

### Food Preservative Formulation

Formulations for the production of red palm oil (RPO) based food preservative were included of food gum, maltodextrin, emulsifiers and RPO. All wall materials were dissolved in warm deionized water (50°C) prior to the addition of RPO, with gentle stirring. Emulsions were prepared at 33% (w/w) total solids with an oil load of 10% (wet basis). Following complete dissolution of coating materials in purified water as the continuous phase and lecithin in RPO as the core material, oil-in-water type emulsion was prepared. All emulsions were prepared in double stages homogenization. Then, spray-drying of the emulsions was carried out using a pilot plant spray dryer (GEA Niro A/S, Soeborg, Denmark). The emulsion was spray dried at a flow rate of 0.5 ml/s. The inlet temperature was set at 180°C and outlet temperature was set at 80 ± 5°C. The resultant microcapsules were collected in a glass jar. Microcapsules were collected and stored in opaque, air tight containers at 4°C prior to further analysis.

### EXAMPLE 7

### Topical Cream Formulation

Preparation of O/W nanoemulsion was prepared according to Zhou et al., 2010 with slight modifications. The formulations were prepared by mixing 5% of surfactant, 15% of oil phase and 80% of water phase. Surfactant was dissolved into the oil. The oil phase was then stirred in the glycerol aqueous solution at 600 rpm for about 10 min. The mixture was emulsified by stirrer at 5000 rpm for 2 min. Subsequently, the pre-emulsion obtained was passed through a high pressure homogenizer (NS1001L, Niro Soavi, Italy) at a pressure of 1,000 bar for 4 cycles. Next, the hot red palm oil (RPO) emulsion was cooled to room temperature. Samples were stored at 25 °C.

### EXAMPLE 8

### Yogurt Formulation

**Table 1 Yogurt Formulation**

| *Ingredient* | *Control* | *15g*/*L ERPO* | *30g*/*L ERPO* | *50g*/*L ERPO* |
|---|---|---|---|---|
| 2% fat milk, L | 1 | 1 | 1 | 1 |
| Nonfat dry milk, g | 50 | 35 | 20 | - |
| Sugar, g | 50 | 50 | 50 | 50 |
| ERPO | - | 15 | 30 | 50 |
| *L. bulgaricus,* mL | 0.26 | 0.26 | 0.26 | 0.26 |
| *S. thermophiles,* mL | 0.26 | 0.26 | 0.26 | 0.26 |
| Banana flavor, mL | 3 | 3 | 3 | 3 |
| Banana puree, g | 50 | 50 | 50 | 50 |

The ingredient and the composition of the yogurt fortified with varying concentrations of red palm oil are shown in Table 1. The ingredients were mixed and heated to 60°C for 1 minute to increase the incorporation of the ingredients before homogenization. The yogurt mix was then homogenised in a two-stage homogenizer at 2,000 PSI for 15 seconds. The mixture was then pasteurized at 85°C for 30 minutes. The mixture was immediately cooled in a water bath to 43°C for inoculation of starter cultures. A starter culture was added and the mixture was incubated at 43°C until the pH drops to 4.5. The yogurt mix was then quickly cooled in an ice bath and stored overnight at 4°C in a cooler.

### EXAMPLE 9

### Antimicrobial Activity

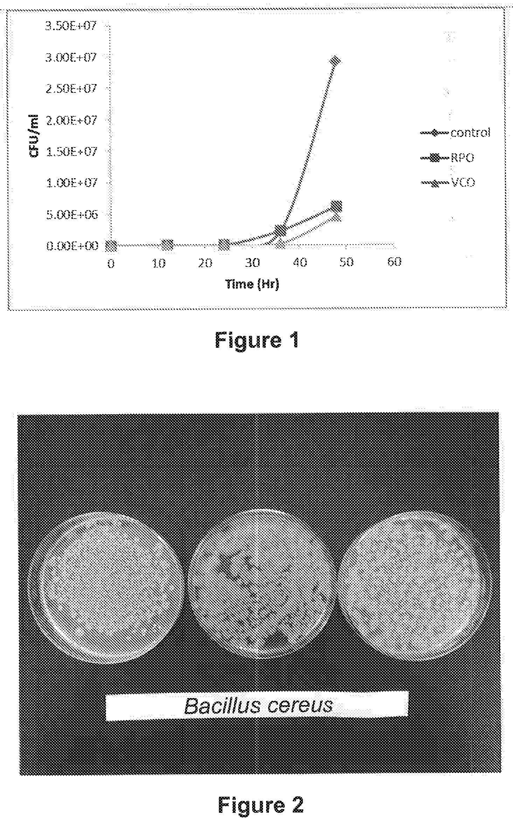

Data of minimum inhibitory concentration of red palm olein (RPO), virgin coconut oil (VCO) and refined, bleached and deodorized palm olein (RBDPO) are tabulated in a graph as shown in Figure 1.

Referring to Figure 1, the total plate count of noodle (cfu/ml) stored at room temperature after 0 hr, 12 hr, 24 hr, 48 hr and 60 hr.

Inhibition of red palm olein (RPO), virgin coconut oil (VCO) and refined, bleached and deodorized palm olein (RBDPO) towards different types of microorganisms are shown in Figures 2, 3, 4 and 5.

Figure 2 shows inhibition of virgin coconut oil (left most petri dish) and red palm oil (right most petri dish) on the growth of *Bacillus cereus.* The middle petri dish is a control.

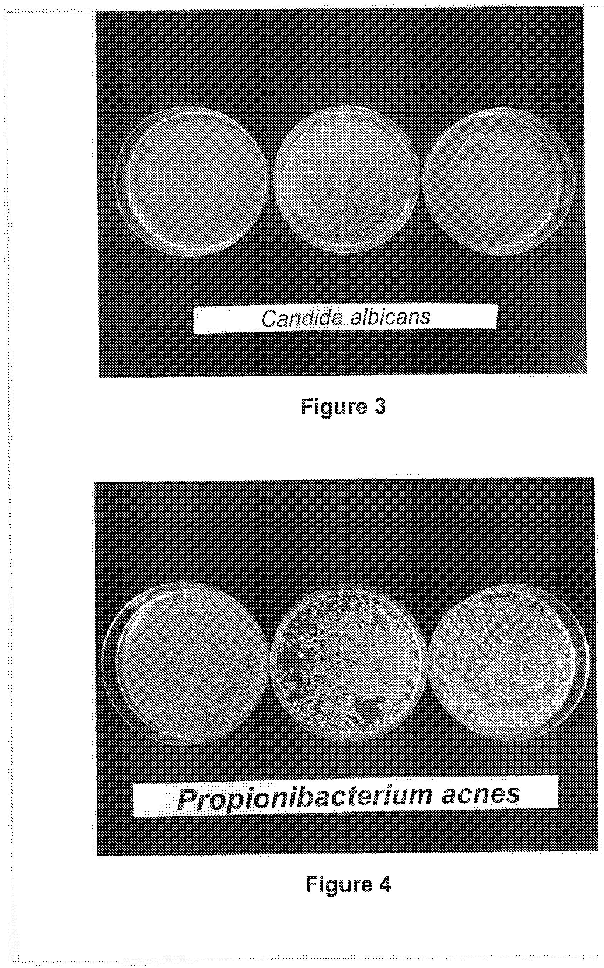

Figure 3 shows inhibition of virgin coconut oil (left most petri dish) and red palm oil (right most petri dish) on the growth of *Candida albicans.* The middle petri dish is a control.

Figure 4 shows inhibition of virgin coconut oil (left most petri dish) and red palm oil (right most petri dish) on the growth of *Propionibacterium acnes.* The middle petri dish is a control.

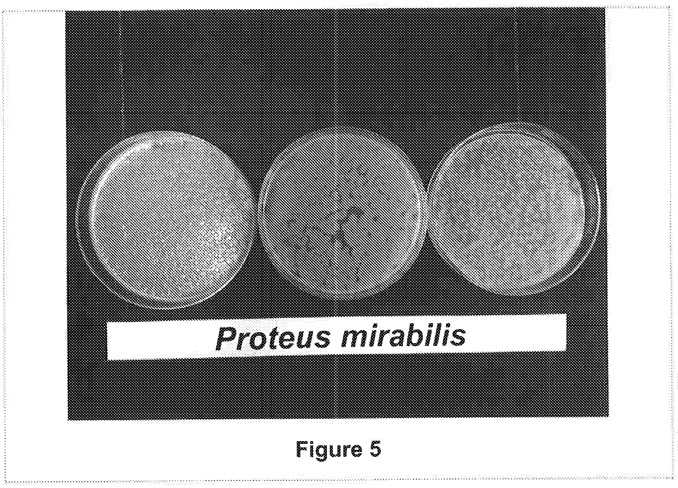

Figure 5 shows inhibition of virgin coconut oil (left most petri dish) and red palm oil (right most petri dish) on the growth of *Proteus mirabilis.* The middle petri dish is a control.

## Claims

1. A composition comprising red palm oil or one or more fractions,
wherein the red palm oil comprises carotenoids, vitamin E, sterols, phosphatides and total alcohols,
wherein the content of carotenoids is in the amount of 500-700 ppm, the content of vitamin E is 500-800 ppm, the content of sterols is up to 300 ppm, the content of phosphatides is 500-100 ppm, and the content of total alcohols is 0-800 ppm.

2. A composition according to claim 1, further comprising coconut oil or one or more of its fractions.

3. A composition according to claim 2, wherein the fraction is a coconut oil concentrate obtained by molecular distillation of the oil.

4. A composition according to claim 3, wherein the fraction is an unsaponifiable fraction contained in a concentrate obtained by molecular distillation of the oil.

5. A composition according to claim 4, wherein the quantities by weight of concentrate of red palm oil and red palm oil vary respectively between 10% and 90% and between 90% and 10% so that the quantities of these oils are 100% in total.

6. A composition according to claim 5, wherein the quantities by weight of the concentrate of red palm oil and of coconut oil are 70% and 30%, respectively.

7. A composition according to any one of the preceding claims, having a total content by weight of red palm oil or its fractions and coconut oil or its fractions in the order of 5%-100%.

8. A composition according to any one of the preceding claims for use in therapy.

## Patentansprüche

1. Zusammensetzung, umfassend rotes Palmöl oder eine oder mehrere Fraktionen,
wobei das rote Palmöl Carotinoide, Vitamin E, Sterine, Phosphatide und Gesamtalkohole umfasst,
wobei der Gehalt an Carotinoiden in der Menge von 500-700 ppm vorliegt, der Gehalt an Vitamin E 500-800 ppm beträgt, der Gehalt an Sterinen bis zu 300 ppm beträgt, der Gehalt an Phosphatiden 500-100 ppm beträgt und der Gehalt an Gesamtalkoholen 0-800 ppm beträgt.

2. Zusammensetzung nach Anspruch 1, ferner umfassend Kokosöl oder eine oder mehrere seiner Fraktionen.

3. Zusammensetzung nach Anspruch 2, wobei es sich bei der Fraktion um ein Kokosölkonzentrat handelt, das durch molekulare Destillation des Öls erhalten wird.

4. Zusammensetzung nach Anspruch 3, wobei es sich bei der Fraktion um eine nicht verseifbare Fraktion handelt, die in einem durch Molekulardestillation des Öls erhaltenen Konzentrat enthalten ist.

5. Zusammensetzung nach Anspruch 4, wobei die Gewichtsmengen an Konzentrat von rotem Palmöl und an rotem Palmöl zwischen 10 % und 90 % bzw. zwischen 90 % und 10 % schwanken, so dass die Mengen dieser Öle insgesamt 100 % ergeben.

6. Zusammensetzung nach Anspruch 5, wobei die Gewichtsmengen des Konzentrats von rotem Palmöl und von Kokosöl 70 % bzw. 30 % betragen.

7. Zusammensetzung nach einem der vorhergehenden Ansprüche, aufweisend einen Gesamtgewichtsgehalt an rotem Palmöl oder seinen Fraktionen und Kokosöl oder seinen Fraktionen in der Größenordnung von 5 % bis 100 %.

8. Zusammensetzung nach einem der vorhergehenden Ansprüche zur therapeutischen Verwendung.

## Revendications

1. Composition comprenant de l'huile de palme rouge ou une ou plusieurs fractions,
dans laquelle l'huile de palme rouge comprend des caroténoïdes, de la vitamine E, des stérols, des phosphatides et des alcools totaux,
dans laquelle la teneur en caroténoïdes est de 500-700 ppm, la teneur en vitamine E est de 500-800 ppm, la teneur en stérols est jusqu'à 300 ppm, la teneur en phosphatides est de 500-100 ppm et la teneur en alcools totaux est de 0-800 ppm.

2. Composition selon la revendication 1, comprenant en outre de l'huile de coco ou une ou plusieurs de ses fractions.

3. Composition selon la revendication 2, dans laquelle la fraction est un concentré d'huile de coco obtenu par distillation moléculaire de l'huile.

4. Composition selon la revendication 3, dans laquelle la fraction est une fraction insaponifiable contenue dans un concentré obtenu par distillation moléculaire de l'huile.

5. Composition selon la revendication 4, dans laquelle les quantités en poids de concentré d'huile de palme rouge et d'huile de palme rouge varient respectivement entre 10 % et 90 % et entre 90 % et 10 % de sorte que les quantités de ces huiles sont de 100% au total.

6. Composition selon la revendication 5, dans laquelle les quantités en poids du concentré d'huile de palme rouge et d'huile de noix de coco sont respectivement de 70 % et 30 %.

7. Composition selon l'une quelconque des revendications précédentes, ayant une teneur totale en poids d'huile de palme rouge ou de ses fractions et d'huile de noix de coco ou de ses fractions de l'ordre de 5 % à 100 %.

8. Composition selon l'une quelconque des revendications précédentes, destinée à être utilisée en thérapie.
